(19) Europäisches Patentamt
European Patent Office
Office européen des brevets

(11) EP 1 091 775 B1

(12) **FASCICULE DE BREVET EUROPEEN**

(45) Date de publication et mention de la délivrance du brevet:
**11.09.2002 Bulletin 2002/37**

(21) Numéro de dépôt: **99926570.5**

(22) Date de dépôt: **30.06.1999**

(51) Int Cl.$^7$: **A61L 27/00**, A61L 31/00

(86) Numéro de dépôt international:
**PCT/FR99/01568**

(87) Numéro de publication internationale:
**WO 00/001428 (13.01.2000 Gazette 2000/02)**

# (54) COMPOSITIONS BIPHASIQUES INJECTABLES, NOTAMMENT UTILES EN CHIRURGIES REPARATRICE ET ESTHETIQUE

INJIZIERBARE ZWEIPHASIGE ZUSAMMENSETZUNGEN FÜR KOSMETISCHE UND PLASTISCHE CHIRURGIE

DIPHASIC INJECTION COMPOSITION, IN PARTICULAR USEFUL IN REPARATIVE AND PLASTIC SURGERY

(84) Etats contractants désignés:
**DE ES FR GB IT**

(30) Priorité: **01.07.1998 FR 9808386**

(43) Date de publication de la demande:
**18.04.2001 Bulletin 2001/16**

(73) Titulaires:
- **CORNEAL INDUSTRIE**
  **74370 Pringy (FR)**
- **Dermatech**
  **92400 Courbevoie (FR)**

(72) Inventeurs:
- **TAUPIN, Valérie**
  **F-92400 Courbevoie (FR)**
- **PIRON, Estelle**
  **F-31270 Villeneuve Tolosane (FR)**
- **THOLIN, Raymonde**
  **F-74000 Annecy (FR)**
- **VILLAIN, Franck**
  **F-74000 Annecy (FR)**

(74) Mandataire: **Le Roux, Martine**
**Cabinet Beau de Loménie**
**158, rue de l'Université**
**75340 Paris Cédex 07 (FR)**

(56) Documents cités:
**EP-A- 0 402 031**
**EP-A- 0 826 381**
**WO-A-96/33751**
**FR-A- 2 568 127**
**FR-A- 2 717 815**
**US-A- 4 563 490**
**US-A- 4 657 553**

- **VAZQUEZ B ET AL: "HYDROGELS BASED ON GRAFT COPOLYMERIZATION OF HEMA/BMA MIXTURES ONTOSOLUBLE GELATIN: SWELLING BEHAVIOUR" POLYMER, vol. 36, no. 11, 1 mai 1995 (1995-05-01), pages 2311-2314, XP000519740**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour principal objet des compositions biphasiques injectables, notamment utiles en chirurgies réparatrice et esthétique. Elle a plus précisément pour objet :

- des compositions biphasiques biocompatibles, comprenant une phase dispersée en suspension dans une phase continue ;
- un procédé de préparation desdites compositions ;
- un matériau de comblement, utile en chirurgie réparatrice et en chirurgie esthétique, à base desdites compositions biphasiques.

La présente invention propose notamment une solution satisfaisante au problème technique du comblement durable des défauts de volume de la peau, tels les rides ou cicatrices, notamment au niveau du visage.
En référence à ce problème technique, il a déjà été proposé, selon l'art antérieur, différentes approches et notamment différentes compositions biphasiques injectables.
Dès 1970, Jaime Planas a eu l'idée de réaliser des particules de silicone, pour les injecter sous la peau.
Il a plus précisément été décrit :

- dans EP-A-0 406 375 : un implant alloplastique, à base d'un solide histocompatible ; ledit solide, pulvérulent, étant constitué de particules solides de diamètre compris en moyenne entre 10 μm et 200 μm, et qui possèdent une surface lisse, exempte d'angle et d'arête. On trouve, à ce jour, sur le marché, un produit, qui correspond à cette demande de brevet. Ledit produit, commercialisé sous la marque Artecoll®, consiste en des microsphères de polyméthacrylate (PMMA), en suspension dans une solution de collagène ;
- dans EP-A-0 466 300 : un gel viscoélastique comprenant une phase gélatineuse (ayant subie une réticulation de faible taux) dispersée dans une phase liquide (n'ayant pas subie de réticulation) ; lesdites deux phases ayant avantageusement été préparées à partir de fibres de Hylan (acide hyaluronique naturel modifié chimiquement in situ dans le but de faciliter son extraction des tissus) ;
- dans US-A-5,137,875 : des solutions ou dispersions injectables de collagène, renfermant de l'acide hyaluronique en solution ;
- dans WO-A-96 33751 : des compositions biphasiques susceptibles de renfermer dans leurs phases continue et dispersée de l'acide hyaluronique ou l'un de ses sels ; ledit acide ou sel intervenant, "relativement" réticulé, sous la forme de fragments, pour constituer la phase dispersée et en solution aqueuse, pas ou très peu réticulé, pour constituer la phase continue. Il est prévu de faire intervenir, dans ladite phase continue, en combinaison ou en lieu et place dudit acide hyaluronique ou de l'un de ses sels, un autre polymère biocompatible choisi parmi les protéines, les polysaccharides et leurs dérivés.

Un produit, du type composition biphasique, serait par ailleurs en évaluation aux Etats-Unis. Il est principalement constitué de billes de silicone, dispersées dans une solution de polyvinylpyrrolidone.
On note incidemment ici que les documents BP-A-826 381. FR-A-2 568 127, US-A-4 657 553 et US-A-4 563 490 ne décrivent pas, eux, de compositions biphasiques.
Les compositions biphasiques, utiles en chirurgies réparatrice et esthétique, se déclinent donc selon de nombreuses variantes et il n'apparaît pas évident d'en mettre une, au point, optimisée en référence aux nombreux paramètres que sont :

- la nature et la forme exactes de la phase continue ;
- les nature, forme, état de surface... de la phase dispersée.

Dans un tel contexte, les Demanderesses proposent un nouveau type de composition biphasique, particulièrement performant.
La composition biphasique biocompatible du type de l'invention comprend donc une phase dispersée en suspension dans une phase continue et, de façon caractéristique, ladite phase dispersée consiste en des particules d'au moins un hydrogel d'un (co)polymère obtenu par polymérisation et réticulation de l'acide acrylique et/ou de l'acide méthacrylique et/ou d'au moins un dérivé desdits acides.
Les compositions biphasiques de l'invention sont des compositions injectables. Elles ont été formulées dans cette optique. C'est notamment à cette fin qu'elles renferment une phase continue ; ladite phase servant de véhicule d'injection aux particules de la phase dispersée.
Le qualificatif injectable, employé dans le présent texte, signifie injectable manuellement au moyen de seringues munies d'aiguilles classiques. Les compositions biphasiques de l'invention sont particulièrement intéressantes

en ce qu'elles peuvent être formulées pour être injectables au moyen d'aiguilles très fines (d'un diamètre compris entre 0,3 et 0,5 mm). L'homme du métier comprend que le paramètre déterminant est celui de la plus grande dimension des particules en suspension. Dans le cadre de la présente invention, il est notamment possible de formuler des compositions injectables au travers d'aiguilles hypodermiques de 30 G ½, 27 G ½, 26 G ½, 25 G. Lesdites compositions constituent la variante la plus avantageuse des compositions de l'invention.

Les compositions biphasiques injectables de l'invention sont tout particulièrement destinées à une injection dermique (superficielle. moyenne ou profonde) pour une implantation dans le derme. A cet effet, dans le but d'éliminer toute sensation désagréable voire toute douleur au cours de leur injection et pendant leur implantation, elles sont avantageusement tamponnées à un pH compris entre 6,5 et 7,5, de préférence compris entre 7 et 7,4, de façon encore plus préférée entre 7,2 et 7,3.

Ainsi, les deux phases continue et dispersée desdites compositions biphasiques sont-elles avantageusement tamponnées à ce pH.

On met généralement en oeuvre un tampon phosphate.

Les compositions biphasiques de l'invention comportent donc une phase dispersée, originale, telle que précisée ci-dessus, en suspension dans une phase continue adéquate.

Ladite phase continue doit, en effet, être à même d'assurer plusieurs fonctions, et notamment :

a) elle doit maintenir en suspension, de façon stable, la phase dispersée ;
b) elle doit constituer un véhicule d'injection performant ;
c) après injection et implantation de la composition biphasique, elle doit avantageusement protéger ladite phase dispersée (notamment l'empêcher de migrer et favoriser la formation de fibroblastes autour de ses particules, ce qui en ralentit la dégradation).

En référence aux points a et b, on comprend qu'il faut adopter un compromis. Ladite phase continue doit être suffisamment fluide pour pouvoir aisément être injectée et suffisamment visqueuse pour éviter la décantation de la phase dispersée.

Le compromis évoqué ci-dessus peut être obtenu avec différents types de phase continue. Dans le cadre de l'invention, certains types, précisés ci-après, sont largement préférés.

On se propose maintenant de fournir des précisions sur chacune des phases - phase continue, phase dispersée - des compositions biphasiques de l'invention.

A titre de phase continue, on fait intervenir une solution aqueuse d'au moins un polymère choisi parmi les protéines, les polysaccharides et leurs dérivés, réticulé. Ledit polymère , selon sa nature, pour assurer les fonctions rappelées ci-dessus est susceptible d'intervenir faiblement réticulé ou fortement réticulé. On peut notamment faire intervenir, comme protéine : le collagène, l'albumine, l'élastine ...

comme polysaccharide ou dérivé de polysaccharide : l'acide hyaluronique, ses sels, les sulfates de chondroïtine, de kératane, l'héparine, l'acide alginique, l'amidon, la carboxyméthylcellulose, le chitosane.

La réticulation de ce type de polymère ne pose pas de difficultés particulières à l'homme du métier.

On préconise tout particulièrement de faire intervenir "une solution aqueuse" d'un polymère choisi parmi l'acide hyaluronique, ses sels et mélanges de ses sels; ledit polymère étant réticulé. En fait, selon une variante préférée, ladite phase continue des compositions biphasiques de l'invention est un hydrogel d'un polymère réticulé choisi, parmi l'acide hyaluronique, ses sels et mélanges de ses sels; ledit polymère réticulé consistant avantageusement en un hyaluronate de sodium.

On emploie dans la suite du présent texte le terme acide hyaluronique comme nom générique pour désigner aussi bien l'acide hyaluronique per se que ses sels ou mélanges de sels et notamment les sels de hyaluronate. Les compositions biphasiques de l'invention renferment avantageusement, dans leur phase continue, à titre de polymère choisi parmi l'acide hyaluronique, ses sels et mélanges de ses sels, du hyaluronate de sodium. On précise déjà que ledit hyaluronate de sodium intervenant est avantageusement d'origine bactérienne.

Ledit acide hyaluronique (ou au moins l'un de ses sels) a été plus spécialement retenu au vu de ses propriétés avantageuses. Il peut notamment être obtenu par voie bactérienne, par voie cellulaire (donc exempt de tout contaminant de type virus ou prions). Il présente à la fois un fort caractère gélatineux, un pouvoir lubrifiant appréciable, une bonne biocompatibilité et une bonne tenue dans l'organisme. Il est par ailleurs aisément réticulable.

Réticulé, il est susceptible de présenter la viscosité requise dans le contexte de l'invention et, en tout état de cause, il est plus résistant à la dégradation et à la chaleur (ce dernier point n'est pas négligeable dans la mesure où les compositions de l'invention sont généralement stérilisées à l'autoclave).

De façon caractéristique, la phase continue des compositions de l'invention est avantageusement à base d'acide hyaluronique réticulé. Il ne s'agit plus vraiment d'une solution aqueuse mais d'un hydrogel. Ledit acide hyaluronique a été réticulé, de façon classique, à l'aide d'au moins un agent réticulant. On préconise, pour l'obtention d'un hydrogel renfermant une quantité raisonnable dudit agent réticulant, d'utiliser comme matériau de départ un acide

hyaluronique dont la masse moléculaire est supérieure ou égale à 1 million de Daltons. Selon une variante avantageuse, on préconise d'utiliser un acide hyaluronique dont la masse moléculaire est comprise entre 2 et 4 millions de Daltons. On préconise par ailleurs de mettre en oeuvre ladite réticulation, via les fonctions hydroxy de l'acide hyaluronique, au moyen d'un agent réticulant (au moins un), dans des conditions qui conduisent à un taux de réticulation dudit acide hyaluronique (matériau de départ) caractérisé par le rapport : nombre total de fonctions réactives dudit agent réticulant / nombre total de motifs disaccharidiques des molécules d'acide hyaluronique présentes compris entre 0,25 et 0,50.

En fait, le réseau de l'hydrogel, qui constitue ainsi la phase continue des compositions biphasiques de l'invention, est à base de molécules d'acide hyaluronique reliées par des ponts de molécules d'agent réticulant ; chacun des motifs disaccharidiques desdites molécules d'acide hyaluronique ayant avantageusement entre 0,25 et 0,50 de ses fonctions hydroxy engagée dans de tels ponts.

A titre d'agent réticulant, on peut faire intervenir tout agent connu pour réticuler l'acide hyaluronique par l'intermédiaire de ses fonctions hydroxy - agent réticulant au moins bifonctionnel - et notamment un polyépoxyde ou ses dérivés. A titre de tel agent réticulant, on peut notamment faire intervenir l'épichlorhydrine, le divinylsulfone, le 1,4-bis (2,3-époxypropoxy)butane (ou 1,4-bis(glycidyloxy)butane ou encore 1,4-butanediol diglycidyl ether = BDDE), le 1,2-bis (2.3-époxypropoxy)éthylène, le 1-(2,3-époxypropyl)-2,3-époxy cyclohexane ... Il n'est pas exclu du cadre de l'invention de faire intervenir plusieurs agents réticulants ... On préconise tout particulièrement de faire intervenir le 1,4-butanediol diglycidyl éther (BDDE).

L'homme du métier sait, en tout état de cause, maîtriser la réticulation de l'acide hyaluronique.

L'hydrogel constitutif de la phase continue des compositions biphasiques de l'invention, à base d'acide hyaluronique réticulé, renferme avantageusement ledit acide hyaluronique réticulé à une concentration comprise entre 10 et 25 mg/g, avantageusement entre 15 et 25 mg/g. De façon nullement limitative, on peut préciser ici que ledit hydrogel renferme en fait généralement plus de 95 % en poids d'eau ...

On rappelle incidemment ici que l'acide hyaluronique, à partir duquel l'hydrogel constitutif de la phase continue des compositions biphasiques de l'invention est de préférence élaboré, est avantageusement obtenu par voie bactérienne (plutôt que par extraction à partir de tissus d'animaux, crêtes de coq et cordons ombilicaux notamment ...) et que l'on préconise tout particulièrement l'intervention d'un hyaluronate de sodium. On préconise, en fait, tout particulièrement, pour l'élaboration dudit hydrogel, l'intervention de fibres de hyaluronate de sodium, obtenues par voie bactérienne.

La phase dispersée consiste en des particules d'au moins un hydrogel d'un (co)polymère obtenu par polymérisation et réticulation de l'acide acrylique et/ou de l'acide méthacrylique et/ou d'au moins un dérivé desdits acides.

Lesdites particules ont classiquement leur taille plus ou moins définie, dans la mesure où :

- elles ne doivent pas être trop petites : dans une telle hypothèse, elles seraient rapidement éliminées par les cellules géantes ou les macrophages, elles migreraient trop aisément et pourraient ainsi développer un effet carcinogène ; et
- ne doivent pas, non plus, être trop volumineuses : dans une telle hypothèse, on rencontrerait des difficultés à les injecter au travers d'aiguilles hypodermiques (telles des aiguilles 30 G ½ qui présentent un diamètre interne de 160 µm) et elles pourraient ne pas convenir pour combler des rides de petite taille... On note ici que, pour ce qui concerne la taille maximale desdites particules, on prendra en compte leur déformabilité, plus réduite, au vu du matériau les constituant, que celle de particules d'autres types.

Généralement, lesdites particules ont leur plus grande dimension comprise entre 10 et 120 µm, avantageusement entre 20 et 80 µm.

On parle de la plus grande dimension desdites particules (i.e de leur diamètre équivalent) dans la mesure où il s'agit avantageusement de fragments d'hydrogel qui présentent une surface rugueuse, fragments d'hydrogel généralement obtenus par broyage d'une masse d'hydrogel.

Il n'est nullement, expressément, exclu du cadre de l'invention de faire intervenir des particules à symétrie de révolution, présentant une surface lisse, exempte d'angle et d'arête mais on préfère, de loin, l'intervention de fragments tels que précisés ci-dessus, fragments que l'on peut obtenir aisément et qui, a priori, présentent deux avantages. En effet :

- une surface lisse d'implant a plus de risques de provoquer la formation de tumeurs qu'une surface rugueuse (*Prog. Exp. Tumor Res.*. vol. 5, pp. 85-133 : "Carcinogenesis through solid state surfaces", par F. Bischoff et G. Bryson) ;
- une surface granuleuse de particules favorise la croissance des tissus fibreux autour, fixant ainsi les particules sur le site d'injection et évitant leur migration (*Cosmetics*, vol. 100, N°6, pp. 1570-1574 : "Bioplastique at 6 years", par Ersek R.A., Gregory S.R. et Salisbury M.D.).

Les fragments constituant la phase dispersée des compositions de l'invention peuvent en fait présenter des formes totalement aléatoires et notamment des géométries ovales, arrondies, triangulaires, carrées ... voire une forme de bâtonnets...

Lesdites particules sont des particules d'hydrogel. En cela, elles sont notamment moins traumatisantes que des particules, type PMMA, de l'art antérieur.

Généralement, lesdites particules ont, à l'équilibre, une teneur en eau comprise entre 10 et 40 % en poids, avantageusement voisine de 25 % en poids,

Ledit hydrogel est un hydrogel d'un polymère ou copolymère hydrophile réticulé méthacrylique et/ou acrylique. Il est obtenu par polymérisation et réticulation d'au moins un monomère choisi parmi l'acide acrylique, l'acide méthacrylique et leurs dérivés.

Ledit hydrogel est avantageusement obtenu à partir d'au moins un monomère choisi parmi :

| | |
|---|---|
| l'acide acrylique<br>l'acrylate d'éthyle<br>l'acrylate de propyle<br>l'acrylate de n-butyle<br>l'acrylate d'isobutyle<br>l'acrylate d'hexyle<br>l'acrylate d'octyle<br>l'acrylate de n-décyle<br>l'acrylate de dodécyle<br><br>le méthacrylate d'hydroxyéthyle (HEMA)<br>le méthacrylate d'hydroxypropyle<br>le méthacrylate d'hydroxybutyle<br>le méthacrylate d'hydroxyisobutyle<br>le méthacrylate d'hydroxyhexyle<br>le méthacrylate d'hydroxyoctyle<br>le méthacrylate d'hydroxy-n-décyle<br>le méthacrylate d'hydroxydodécyle. | l'acide méthacrylique<br>le méthacrylate de méthyle (MMA)<br>le méthacrylate d'éthyle (EMA)<br>le méthacrylate de propyle<br>le méthacrylate de n-butyle<br>le méthacrylate d'isobutyle<br>le méthacrylate d'hexyle<br>le méthacrylate d'octyle<br>le méthacrylate de n-décyle<br>le méthacrylate de dodécyle |

On comprend bien évidemment que, dans la mesure où ledit (co)polymère (méth)acrylique doit être hydrophile (pour constituer ledit hydrogel), il est exclu qu'il s'agisse d'un polyméthacrylate de méthyle (PMMA). Lorsque ledit méthacrylate de méthyle (MMA) intervient, il intervient forcément à titre de comonomère.

Le (co)polymère hydrophile (méth)acrylique, constitutif des particules de la phase dispersée des compositions biphasiques de l'invention, consiste avantageusement en du polyméthacrylate d'hydroxyéthyle (PHEMA) réticulé ou, encore plus avantageusement, en un copolymère réticulé :

- de méthacrylate d'hydroxyéthyle (HEMA) et
- de méthacrylate d'éthyle (EMA).

Ledit méthacrylate d'hydroxyéthyle (HEMA) confère aux particules de la phase dispersée hydrophilie et souplesse tandis que ledit méthacrylate d'éthyle (EMA) optimise leurs propriétés mécaniques. La quantité d'intervention dudit EMA doit évidemment rester dans des limites raisonnables pour ne pas compromettre le caractère hydrophile du copolymère. On précise ici que ledit copolymère est avantageusement obtenu par copolymérisation, pour 100 parties en poids de monomères : HEMA+EMA, de 77,5 à 87,5 parties en poids (avantageusement, de 80 à 85 parties en poids) d'HEMA et de 12,5 à 22,5 parties en poids (avantageusement, de 15 à 20 parties en poids) d'EMA. Selon une variante particulièrement avantageuse, il est obtenu par copolymérisation de 82,5 parties en poids d'HEMA et de 17,5 parties en poids d'EMA.

Ainsi, le copolymère méthacrylique qui constitue les particules de la phase dispersée des compositions biphasiques de l'invention renferme-t-il des motifs [HEMA] et des motifs [EMA] dans un rapport R : $R = \frac{[HEMA]}{[EMA]}$, généralement compris entre 3,0 et 6,1 , avantageusement compris entre 3,5 et 5 et valant, selon une variante préférée, 4,1.

Ledit copolymère poly[HEMA/EMA] est, comme précisé ci-dessus, réticulé. Une telle réticulation est indispensable pour assurer la cohésion du matériau et sa stabilité. Un (au moins un) agent de réticulation - bifonctionnel - doit donc intervenir, en une quantité efficace, lors de la copolymérisation des monomères HEMA et EMA. Cette quantité efficace - généralement, au maximum de quelques parties en poids : en principe comprise entre 0,5 et 5 parties en

poids, avantageusement comprise entre 0,5 et 2 parties en poids, pour 100 parties en poids de monomères : HEMA + EMA - doit évidemment rester raisonnable. Il ne s'agit pas que l'agent de réticulation intervenant constitue un comonomère et modifie de façon conséquente les propriétés, notamment mécaniques, du copolymère poly(HEMA/EMA).

En tout état de cause, l'homme du métier n'ignore pas que l'augmentation du taux d'agent de réticulation intervenant diminue la teneur en eau des hydrogels et augmente leur température de transition vitreuse.

On indique ici que ledit agent de réticulation intervient dans la structure du copolymère, généralement en une quantité telle, que le rapport

$$R' = \frac{\textit{nombre total de fonctions réactives dudit agent de réticulation présent}}{\textit{nombre total de fonctions réactives (méthacrylates) des réactifs présents (HEMA, EMA)}}$$

soit compris entre $6.10^{-3}$ et $60.10^{-3}$. Ledit rapport R' vaut avantageusement $10^{-2}$.

Pour ce qui concerne les fonctions réactives dudit agent de réticulation. il s'agit avantageusement de fonctions acrylates et/ou méthacrylates. L'homme du métier connaît de nombreux agents de réticulation, porteurs de telles fonctions. et notamment :

les diméthacrylate et diacrylate de butanediol,
les diméthacrylate et diacrylate d'hexanediol,
les diméthacrylate et diacrylate de décanediol,
le diméthacrylate d'éthylène glycol (EDMA),
le diméthacrylate de tétraéthylène glycol.

Dans le cadre de la présente invention, on préconise, de façon nullement limitative, l'intervention des agents de réticulation listés ci-dessus et tout particulièrement celle de l'EDMA.

Ainsi, le copolymère poly(HEMA/EMA), constitutif des particules de la phase dispersée des compositions biphasiques de l'invention, est-il réticulé par des agents de réticulation de ce type (ou d'un type équivalent) dont on trouve évidemment trace dans son squelette.

Ledit copolymère poly(HEMA/EMA) est préparé, de façon connue en soi, par copolymérisation d'un mélange de monomères HEMA et EMA en présence de quantités efficaces d'au moins un initiateur de polymérisation et d'au moins un agent de réticulation.

On a vu ci-dessus qu'en ce qui concerne l'agent de réticulation, il intervient une quantité efficace (généralement de 0,5 à 5 parties en poids et avantageusement de 0,5 à 2 parties en poids, pour 100 parties en poids de monomères : HEMA+EMA), d'un agent de réticulation, tel l'EDMA. Ledit EDMA peut notamment intervenir à raison de 0,8 partie en poids. D'autres agents de réticulation. comme indiqué ci-dessus, peuvent intervenir en lieu et place dudit EDMA.

A titre d'initiateur de la copolymérisation radicalaire HEMA-EMA, on peut notamment utiliser :

- un mélange phosphite de sodium et phosphate de sodium (ou tout autre couple d'oxydo-réduction);
- un composé azo, tel l'azobisisobutyronitrile (AIBN) ou le (2,2'-azobis (2,4-diméthyl valéronitrile)(AIVN), notamment commercialisé par la société WAKO sous la référence V65, dont on reproduit ci-après les formules développées :

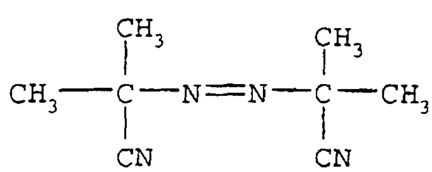

AIBN

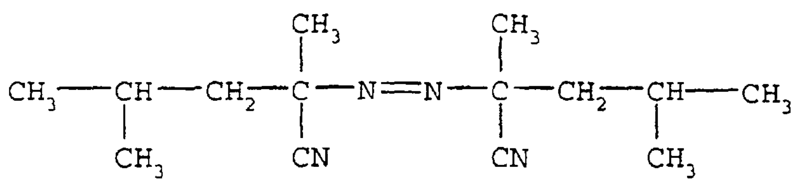

AIVN

Ce dernier composé est particulièrement préféré au vu de sa faible toxicité, ainsi que de celle de ses produits de dégradation. (On notera toutefois, de manière générale, que ledit initiateur de polymérisation intervient en très faible quantité et se trouve généralement éliminé à l'issue du procédé de préparation de l'hydrogel) ;

- un peroxyde, tel le peroxyde de benzoyle.

L'homme du métier sait maîtriser la quantité d'intervention dudit initiateur de polymérisation radicalaire (généralement moins de 1 partie en poids, pour 100 parties en poids de monomères : HEMA+EMA) et de manière générale la cinétique de polymérisation du mélange réactionnel. Il sait notamment que, l'oxygène neutralisant l'action dudit initiateur de polymérisation, il est vivement préférable de l'éliminer du mélange réactionnel avant la montée en température. Un bullage de gaz inerte dudit mélange réactionnel est vivement préconisé. Pour ce qui concerne le programme de chauffe, son optimisation est à la portée de l'homme du métier.

Ledit copolymère réticulé poly(HEMA + EMA) est donc largement préconisé à titre de matériau constitutif des particules de la phase dispersée des compositions biphasiques de l'invention.

Les précisions données ci-dessus en référence à la préparation dudit copolymère, notamment celles relatives à la nature des agent(s) de réticulation et initiateur(s) de polymérisation susceptibles d'intervenir, s'applique bien évidemment au contexte de la (co)polymérisation et réticulation de monomères d'autres natures pour la préparation d'autres polymères ou copolymères convenant pour constituer les particules de la phase dispersée.

Au sein des compositions biphasiques de l'invention, les particules de la phase dispersée interviennent généralement à raison de 10 à 30 % en masse, avantageusement à raison de 15 à 25 % en masse. On a donc généralement :

$$10\ \% \leq \frac{\textit{masse de la phase dispersée}}{\textit{masse de la phase dispersée + masse de la phase continue}} \leq 30\ \%.$$

La phase continue est considérée hydratée, tandis que la phase dispersée peut être considérée sèche ou à l'équilibre. Avantageusement, dans ce type de rapport, on considère la masse sèche de la phase dispersée, i.e la masse sèche des particules.

On précise ici qu'il intervient généralement au sein de la phase dispersée un unique type de particules. Il n'est toutefois nullement exclu du cadre de l'invention de faire intervenir conjointement des particules de forme et/ou nature différentes ...

Pour ce qui concerne la préparation des compositions biphasiques de l'invention, l'homme du métier a déjà compris qu'elle ne soulève pas de difficultés particulières. Ladite préparation, qui constitue le second objet de la présente invention, comprend :

- la préparation de la phase continue (avantageusement celle d'un hydrogel d'acide hyaluronique réticulé) ;
- la préparation de la phase dispersée (des particules d'au moins un hydrogel d'un (co)polymère obtenu par polymérisation et réticulation de l'acide acrylique et/ou de l'acide méthacrylique et/ou d'au moins un dérivé desdits acides) ;
- l'incorporation et le mélange dans ladite phase continue de ladite phase dispersée.

La préparation de la phase continue et notamment celle d'un hydrogel d'acide hyaluronique réticulé, avantageusement au taux de réticulation et à la concentration en acide précisés ci-dessus, ne soulève aucune difficulté particulière.

De la même façon, les particules de la phase dispersée peuvent être obtenues par toute méthode connue en elle-même. Des particules à symétrie de révolution, à surface lisse, et notamment des microsphères, peuvent être obtenues par émulsification.

On a vu que l'on préconisait de préparer des fragments à surface rugueuse ; lesdits fragments étant avantageusement obtenus par broyage mécanique d'une masse d'hydrogel appropriée.

Dans le cadre du procédé de l'invention, on préconise avantageusement de préparer les particules de la phase dispersée, de les sécher et de les ajouter, sèches, à la phase continue.

La composition biphasique préparée est avantageusement stérilisée pour son stockage. On préconise de la conditionner, avant stérilisation et stockage. Elle est avantageusement conditionnée dans des seringues. Elle se trouve, alors, prête à l'emploi.

Selon son dernier objet, l'invention concerne donc un matériau de comblement, utile en chirurgie réparatrice et en chirurgie esthétique, à base des compositions biphasiques telles que décrites ci-dessus.

Ledit matériau est particulièrement performant, en terme notamment de stabilité et de caractère non traumatisant, de par les nature et consistance de ses phases continue (avantageusement de l'HA réticulé) et dispersée (hydrogel).

On préconise l'utilisation d'un tel matériau de comblement pour combler notamment des rides du visage telles la ride glabellaire, les rides péribuccales, les sillons naso-géniens, pour atténuer les pattes d'oie ...

L'invention est illustrée par l'exemple ci-après.

On a préparé une composition biphasique de l'invention à partir de fibres de hyaluronate de sodium (NaHa) pour la phase continue et de fragments d'un hydrogel poly[HEMA/EMA] pour la phase dispersée.

a) Préparation de la phase continue

- Ladite phase continue est préparée avec des fibres de hyaluronate de sodium (de masse moléculaire : $M_w \approx 2,9\ 10^6$ Da), d'origine bactérienne. Une solution à 11,5 % en masse desdites fibres dans de la soude 0,25 M est tout d'abord préparée.
- A ladite solution homogénéisée, 60 µl de 1,4-butanediol diglycidyl éther (BDDE) sont ajoutés. Le mélange obtenu, homogénéisé, est mis au bain-marie, à 50°C, pendant 2 heures.

Le gel résultant est alors neutralisé par ajout d'acide chlorhydrique 1M, puis dilué par du tampon phosphate à pH 7,2, jusqu'à obtenir une concentration de 20 mg/g en NaHa.

Ce gel est ensuite purifié par dialyse dans un bain de tampon phosphate afin d'éliminer de sa structure à la fois l'agent réticulant (BDDE) et le polymère qui n'ont pas réagi.

Au sein d'un tel gel. le rapport : nombre total de fonctions réactives dudit agent réticulant / nombre total de motifs disaccharidiques des molécules du polymère présentes est de 0.27.

b) Préparation de la phase dispersée

- On a préparé dans un premier temps des palets ou bâtonnets d'un hydrogel acrylique, de la manière suivante.

Dans un bêcher, on a versé 82.5 g de méthacrylate d'hydroxyéthyle (HEMA), 17,5 g de méthacrylate d'éthyle (EMA), 1 g de 4-méthacryloxy-2-hydroxybenzophénone (MOBP), 0,8 g de diméthacrylate d'éthylène glycol (EDMA) et 0.2 g de peroxyde de benzoyle.

On a homogénéisé le mélange réactionnel puis on y a fait buller de l'argon pendant 2 min. La solution ainsi désoxygénée a alors été répartie dans des moules ; lesdits moules étant ensuite placés :

- 48 heures dans un bain-marie à 40°C ;
- 48 heures dans un bain-marie à 60°C ;
- puis 48 heures dans une étuve à 100°C.

Le matériau obtenu, après refroidissement, a été démoulé.

- Les palets ou bâtonnets démoulés ont alors été broyés mécaniquement. La poudre ainsi obtenue a été tamisée successivement sur des tamis de maillage 100, 40 et 25 µm. Seuls les fragments récupérés sur le tamis 25 µm ont été conservés. Ceux-ci ont alors été purifiés dans un bain bouillonnant alcool/eau, laissés décantés pour l'élimination des petits fragments qui seraient restés sur le tamis 25 µm, et enfin rincés dans deux bains successifs d'eau désionisée. Après séchage, sous flux (classe 100), des fragments ainsi obtenus, ceux-ci ont à nouveau été tamisés sur 25 µm, avant d'être utilisés pour le produit final.

c) Préparation de la composition biphasique

11 g de fragments secs, tels qu'obtenus au point b) sont ajoutés au gel réticulé de NaHa, tel qu'obtenu au point a). Le tout est mélangé pour obtenir une dispersion homogène. Le rapport massique m = masse des fragments / (masse des fragments + masse de gel), est de 0,2.

Des échantillons de ladite composition biphasique obtenue ont notamment été soumis à des tests d'extrudabilité, en vue de caractériser la force nécessaire à son injection. Lesdits tests ont été effectués à l'aide d'un appareil de traction VERSATEST (MECMESIN).

Pour une vitesse de compression de 12,5 mm/min, la force caractéristique de l'injection à travers :

- une aiguille de 30 G ½ est de 25 à 30 N,
- une aiguille de 27 G ½ est de 12 à 15 N.

d) Conditionnement de la composition biphasique

La dispersion ou suspension obtenue est mise dans des seringues qui sont stérilisées à l'autoclave. Ladite dispersion est injectable, notamment au travers d'aiguilles de 25 G à 30 G ½.

## Revendications

1. Composition biphasique biocompatible comprenant une phase dispersée en suspension dans une phase continue, **caractérisée en ce que** ladite phase dispersée consiste en des particules d'au moins un hydrogel d'un (co)polymère obtenu par polymérisation et réticulation de l'acide acrylique et/ou de l'acide méthacrylique et/ou d'au moins un dérivé desdits acides ; et

   **en ce que** ladite phase continue est une solution aqueuse d'au moins un polymère, choisi parmi les protéines, les polysaccharides et leurs dérivés, réticulé.

2. Composition selon la revendication 1, **caractérisée en ce que** ladite phase continue est un hydrogel d'un polymère réticulé choisi parmi l'acide hyaluronique, ses sels et mélanges de ses sels, consistant avantageusement en un hyaluronate de sodium.

3. Composition selon la revendication 2, **caractérisée en ce que** l'hydrogel constitutif de ladite phase continue est obtenu à partir dudit polymère dont la masse moléculaire est supérieure ou égale à 1 million de Daltons, avantageusement comprise entre 2 et 4 millions de Daltons, qui a été réticulé, via ses fonctions hydroxy, au moyen d'un agent réticulant, à un taux de réticulation défini par le rapport : nombre total de fonctions réactives dudit agent réticulant / nombre total de motifs disaccharidiques des molécules d'acide hyaluronique présentes compris entre 0,25 et 0,50.

4. Composition selon l'une des revendications 2 ou 3, **caractérisée en ce que** l'hydrogel constitutif de ladite phase continue renferme ledit polymère réticulé à une concentration comprise entre 10 et 25 mg/g, avantageusement entre 15 et 25 mg/g.

5. Composition selon l'une quelconque des revendications 2 à 4, **caractérisée en ce que** ledit polymère choisi parmi l'acide hyaluronique et ses sels a été obtenu par voie bactérienne.

6. Composition selon l'une quelconque des revendications 1 à 5, **caractérisée en ce que** lesdites particules de ladite phase dispersée ont leur plus grande dimension comprise entre 10 et 120 µm, avantageusement entre 20 et 80 µm.

7. Composition selon l'une quelconque des revendications 1 à 6, **caractérisée en ce que** lesdites particules de ladite phase dispersée sont des fragments d'hydrogel qui présentent une surface rugueuse.

8. Composition selon l'une quelconque des revendications 1 à 7, **caractérisée en ce que** lesdites particules de ladite phase dispersée ont une teneur en eau, à l'équilibre, comprise entre 10 et 40 % en poids, avantageusement voisine de 25 % en poids.

9. Composition selon l'une quelconque des revendications 1 à 8, **caractérisée en ce que** ledit (co)polymère est un copolymère réticulé de méthacrylate d'hydroxyéthyle (HEMA) et de méthacrylate d'éthyle (EMA).

10. Composition selon la revendication 9, **caractérisée en ce que** ledit copolymère réticulé renferme les motifs [HEMA] et [EMA] dans un rapport R = $\frac{[HEMA]}{[EMA]}$ ; ledit rapport R étant compris entre 3,0 et 6,1, avantageusement entre 3,5 et 5, et valant selon une variante particulièrement préférée 4,1.

11. Composition selon l'une des revendication 9 ou 10, **caractérisée en ce que** ledit copolymère réticulé est obtenu par réaction, pour 100 parties en poids de monomères : HEMA + EMA, de :

    - 77,5 à 87,5, avantageusement 80 à 85, parties en poids, d'HEMA ; et
    - 12,5 à 22,5, avantageusement 15 à 20, parties en poids, d'EMA ; en présence d'une quantité efficace d'au

moins un agent de réticulation.

**12.** Composition selon l'une quelconque des revendications 1 à 11, **caractérisée en ce qu'**elle renferme de 10 à 30 % en masse, avantageusement de 15 à 25 % en masse, desdites particules en suspension dans ladite phase continue.

**13.** Procédé de préparation d'une composition selon l'une quelconque des revendications précédentes, **caractérisé en ce qu'**il comprend

- la préparation d'une phase continue adéquate ;
- la préparation de particules d'au moins un hydrogel d'un (co)polymère obtenu par polymérisation et réticulation de l'acide acrylique et/ou de l'acide méthacrylique et/ou d'au moins un dérivé desdits acides ; lesdites particules étant avantageusement séchées à l'issue de ladite préparation ;
- l'incorporation et le mélange dans ladite phase continue adéquate desdites particules, avantageusement sèches.

**14.** Procédé selon la revendication 13, **caractérisé en ce que** lesdites particules sont obtenues par broyage mécanique d'une masse d'hydrogel.

**15.** Matériau de comblement, utile en chirurgie réparatrice et en chirurgie esthétique, **caractérisé en ce qu'**il est à base d'une composition biphasique selon l'une quelconque des revendications 1 à 12.

**Patentansprüche**

**1.** Zweiphasige biokompatible Zusammensetzung mit einer in einer kontinuierlichen Phase suspendierten dispersen Phase, **dadurch gekennzeichnet, dass** die disperse Phase aus Teilchen mindestens eines Hydrogels eines durch Polymerisation und Vernetzung von Acrylsäure und/oder Methacrylsäure, und/oder von mindestens einem Derivat dieser Säuren erhaltenen (Co)polymeren besteht, und dadurch, dass die kontinuierliche Phase eine wässrige Lösung von mindestens einem vernetzten Polymeren, ausgewählt aus Proteinen, Polysacchariden und deren Derivaten, ist.

**2.** Zusammensetzung nach Anspruch 1, **dadurch gekennzeichnet, dass** die kontinuierliche Phase ein Hydrogel eines vernetzten Polymers, ausgewählt aus Hyaluronsäure, deren Salzen und Mischungen von deren Salzen ist, das vorteilhaft aus einem Natriumhyaluronat besteht.

**3.** Zusammensetzung nach Anspruch 2, **dadurch gekennzeichnet, dass** das in der kontinuierlichen Phase enthaltene Hydrogel ausgehend von einem Polymer, dessen Molmasse gleich oder größer als 1 Million Dalton, vorteilhaft zwischen 2 und 4 Millionen Dalton, ist, erhalten wird, welches über seine Hydroxylfunktionen mittels eines Vernetzungsmittels zu einem Vernetzungsgrad vernetzt wurde, der durch die Beziehung: Gesamtzahl der reaktiven Funktionen des Vernetzungsmittels / Gesamtzahl der Disaccharideinheiten des Hyaluronsäuremoleküls liegt zwischen 0,25 und 0,50, definiert ist.

**4.** Zusammensetzung nach einem der Ansprüche 2 oder 3, **dadurch gekennzeichnet, dass** das in der kontinuierlichen Phase enthaltene Hydrogel das vernetzte Polymer in einer Konzentration zwischen 10 und 25 mg/g, vorteilhaft zwischen 15 und 25 mg/l, umfasst.

**5.** Zusammensetzung nach einem der Ansprüche 2 bis 4, **dadurch gekennzeichnet, dass** das Polymer aus Hyaluronsäure und ihren Salzen ausgewählt ist und auf bakteriellem Weg erhalten wurde.

**6.** Zusammensetzung nach einem der Ansprüche 1 bis 5, **dadurch gekennzeichnet, dass** die Teilchen der dispersen Phase ihre größte Abmessung zwischen 10 und 120 µm, vorteilhaft zwischen 20 und 80 µm, haben.

**7.** Zusammensetzung nach einem der Ansprüche 1 bis 6, **dadurch gekennzeichnet, dass** die Teilchen der dispersen Phase Bruchstücke eines Hydrogels sind, die eine raue Oberfläche aufweisen.

**8.** Zusammensetzung nach einem der Ansprüche 1 bis 7, **dadurch gekennzeichnet, dass** die Teilchen der dispersen Phase im Gleichgewicht einen Wassergehalt zwischen 10 und 40, vorteilhaft bei 25 Gewichtsprozent, haben.

**9.** Zusammensetzung nach einem der Ansprüche 1 bis 8, **dadurch gekennzeichnet, dass** das (Co)polymer ein vernetztes Copolymer aus Hydroxyethylmethacrylat (HEMA) und Ethylmethacrylat (EMA) ist.

**10.** Zusammensetzung nach Anspruch 9, **dadurch gekennzeichnet,** dass das vernetzte Copolymer die Einheiten [HEMA] und [EMA] im Verhältnis R = [HEMA]/[EMA] umfasst, wobei R zwischen 3,0 und 6,1, vorteilhaft zwischen 3,5 und 5 liegt und bei einer besonders bevorzugten Ausführungsform 4,1 beträgt.

**11.** Zusammensetzug nach einem der Ansprüche 9 oder 10, **dadurch gekennzeichnet, dass** das vernetzte Polymer durch Reaktion von

- 77,5 bis 87,5, vorteilhaft 80 bis 85 Gewichtsteile HEMA und
- 12,5 bis 22,5, vorteilhaft 15 bis 20 Gewichtsteile EMA auf 100 Gewichtsteile Monomer: HEMA + EMA in Gegenwart einer wirksamen Menge eines Vernetzungsmittels erhalten wird.

**12.** Zusammensetzung nach einem der Ansprüche 1 bis 11, **dadurch gekennzeichnet, dass** sie 10 bis 30, vorteilhaft 15 bis 25 Massenprozent der in der kontinuierlichen Phase suspendierten Teilchen enthält.

**13.** Verfahren zur Herstellung einer Zusammensetzung nach einem der vorangehenden Ansprüche, **dadurch gekennzeichnet, dass** es umfasst:

- die Herstellung einer geeigneten kontinuierlichen Phase;
- die Herstellung von Teilchen aus mindestens einem Hydrogel eines durch Polymerisation und Vernetzung von Acrylsäure und/oder Methacrylsäure und/oder von mindestens einem Derivat dieser Säuren erhaltenen (Co) polymeren, wobei die Teilchen am Ende der Herstellung vorteilhaft getrocknet werden;
- Zufügen und Einmischen der vorteilhaft trockenen Teilchen in die geeignete kontinuierliche Phase.

**14.** Verfahren nach Anspruch 13, **dadurch gekennzeichnet**, dass die Teilchen durch mechanisches Zerkleinern eines Hydrogelkörpers erhalten werden.

**15.** In der wiederherstellenden und kosmetischen Chirurgie verwendbares Füllmaterial, **dadurch gekennzeichnet, dass** es auf einer zweiphasigen Zusammensetzung nach einem der Ansprüche 1 bis 12 basiert.

## Claims

**1.** A biocompatible diphasic composition comprising a dispersed phase in suspension in a continuous phase, **characterized in that** said dispersed phase consists of particles of at least one hydrogel of a (co)polymer obtained by polymerizing and cross-linking acrylic acid and/or methacrylic acid and/or at least one derivative of said acids; and **in that** said continuous phase is an aqueous solution of at least one polymer selected from proteins, polysaccharides and their derivatives, cross-linked.

**2.** The composition according to claim 1, **characterized in that** said continuous phase is a hydrogel of a cross-linked polymer selected from hyaluronic acid, its salts and mixtures of its salts, advantageously consisting of a sodium hyaluronate.

**3.** The composition according to claim 2, **characterized in that** the hydrogel constituting said continuous phase is obtained from said polymer whose molecular mass is greater than or equal to 1 million Daltons, advantageously included between 2 and 4 million Daltons, which was cross-linked, *via* its hydroxy functions, by means of a cross-linking agent, at a cross-linking rate defined by the ratio: total number of reactive functions of said cross-linking agent/total number of disaccharide repeating units of the molecules of hyaluronic acid present, included between 0.25 and 0.50.

**4.** The composition according to one of claims 2 or 3, **characterized in that** the hydrogel constituting said continuous phase contains said cross-linked polymer at a concentration included between 10 and 25 mg/g, advantageously between 15 and 25 mg/g.

**5.** The composition according to any one of claims 2 to 4, **characterized in that** said polymer selected from hyaluronic acid and its salts was obtained by the bacterial route.

**6.** The composition according to any one of claims 1 to 5, **characterized in that** said particles of said dispersed phase have their largest dimension included between 10 and 120 μm, advantageously between 20 and 80 μm.

**7.** The composition according to any one of claims 1 to 6, **characterized in that** said particles of said dispersed phase are fragments of hydrogel which present a rough surface.

**8.** The composition according to any one of claims 1 to 7, **characterized in that** said particles of said dispersed phase have a water content, at equilibrium, included between 10 and 40 % by weight, advantageously close to 25% by weight.

**9.** The composition according to any one of claims 1 to 8, **characterized in that** said (co)polymer is a cross-linked copolymer of hydroxyethyl methacrylate (HEMA) and of ethyl methacrylate (EMA).

**10.** The composition according to claim 9, **characterized in that** said cross-linked copolymer contains the repeating [HEMA] and [EMA] units in a ratio $R = \frac{[HEMA]}{[EMA]}$; said ratio R being included between 3.0 and 6.1, advantageously between 3.5 and 5, and, according to a particularly preferred variant, being 4.1.

**11.** The composition according to one of claims 9 or 10, **characterized in that** said cross-linked copolymer is obtained by reaction, for 100 parts by weight of monomers: HEMA + EMA, of:

- 77.5 to 87.5, advantageously 80 to 85, parts by weight of HEMA, and
- 12.5 to 22.5, advantageously 15 to 20, parts by weight of EMA; in the presence of an efficient quantity of at least one cross-linking agent.

**12.** The composition according to any one of claims 1 to 11, **characterized in that** it contains from 10 to 30% by mass, advantageously from 15 to 25% by mass, of said particles in suspension in said continuous phase.

**13.** A method for preparing a composition according to any one of the preceding claims, **characterized in that** it comprises:

- the preparation of an adequate continuous phase;
- the preparation of particles of at least one hydrogel of a (co)polymer obtained by polymerizing and cross-linking acrylic acid and/or methacrylic acid and/or at least one derivative of said acids; said particles being advantageously dried at the end of said preparation;
- the incorporation and the mixture in said adequate continuous phase of said particles, advantageously dried.

**14.** The method according to claim 13, **characterized in that** said particles are obtained by mechanically crushing a mass of hydrogel.

**15.** A filling material, useful in reparative surgery and in plastic surgery, **characterized in that** it is based on a diphasic composition according to any one of claims 1 to 12.